# DEMANDE DE BREVET EUROPEEN

(11) **EP 1 493 995 A1**
(43) Date de publication de la demande: **05.01.2005**
(21) Numéro de dépôt: 04370021.0
(22) Date de dépôt: 02.07.2004
(51) Int. Cl.: G01C 22/00, G01N 3/08

(54) **Dispositif de chargement automatique de masse pour oedomètre à chargement par l'avant**

(30) Priorité: 03.07.2003 FR 0308109
(71) Demandeur: Velleman, Marcel, 59000 Lille (FR)
(72) Inventeur: Velleman, Marcel, 59000 Lille (FR)

(57) **Abrégé**

L'invention concerne un dispositif de chargement automatique de masse pour oedomètre a chargement par l'avant caractérisé en ce qu'il comprend des moyens de chargement / déchargement de masses (15) logées dans un panier (14) sur un plateau (19) relié au bras de l'oedomètre

Application à l'automatisation du chargement des masses pour un oedomètre à chargement par l'avant.

## Description

L'invention concerne le domaine des oedomètres à chargement par l'avant.

L'invention concerne un dispositif permettant de déposer mes masses automatiquement sans intervention humaine et en séquence sur un ensemble de tige et plateau (19) de façon a appliquer une force ou une traction sur l'élément auquel le dispositif est raccordé (1).

Pour l'instant ce type d'oedométre est utilisé de façon manuelle et consiste à poser des poids successifs sur un réceptacle ou plateau (19)

La procédure imposée par la norme actuelle oblige notamment à être présent au moment de la mesure et de manipuler des masses qui vont de 0.25 Kg à 32 Kg , ce qui rend pénible, voire dangereux ce type de manipulation.

Le système concernant la demande de brevet permet de palier à ces inconvénients, sachant que les poids ne sont plus manipulés par l'opérateur mais par le dispositif selon l'invention.

Le système concernant la présente demande ne nécessite pas la présence d'un opérateur après que celui-ci ait lancé le processus automatisé par l'intermédiaire du calculateur et du programme développé pour le faire fonctionner.

Le dispositif selon l'invention est particulièrement destiné en l'automatisation des oedométres à chargement par l'avant (1)

L'invention concerne un dispositif de chargement automatique de masse pour oedomètre a chargement par l'avant caractérisé en ce qu'il comprend des moyens de chargement / déchargement de masses (15) empilées et logées dans un panier (14) sur un plateau (19) relié au bras de l'oedomètre

Selon des modes de réalisation particuliers :
- les moyens de chargement/déchargement comprennent un corps (7) guidé par des tiges parallèle (8) en coulissement entraîné par une vis sans fin (9) et un moteur (10), le dit corps supportant le panier des masses (14) par des câbles (6) ou des tiges centrés par rapport a l'axe
- le plateau tige (19) traverse le panier (14) et les masses (15) de façon libre et en position centrée.
- en ce qui concerne le panier (14), comprend un arrangement de masses (15) , chaque masse présentant des protubérances latérales (17) , lesdites protubérances coopérant chacune avec des moyens de butée (16) notamment en forme évasée situées sur les cotés du panier de support des masses (14).
- lesdits moyens de butées (16) affectés a une première masse sont décalés horizontalement et verticalement par rapport au dit moyen de butée affectés a une seconde masse voisine haute ou basse afin de ne pas gêner l'empilement des masses (15)
- chaque butée (16) présente une forme envasée à pans inclinés tridimensionnelle pour recentrer les masses (15) dans un plan donné lors du déchargement des masses par rapport à l'axe.
- le moteur (10) est commandé par un calculateur géré par un programme avec des fonction d'empilement de masses (15) prédéterminé dans le temps et se rapportant a une norme ou a une méthode.

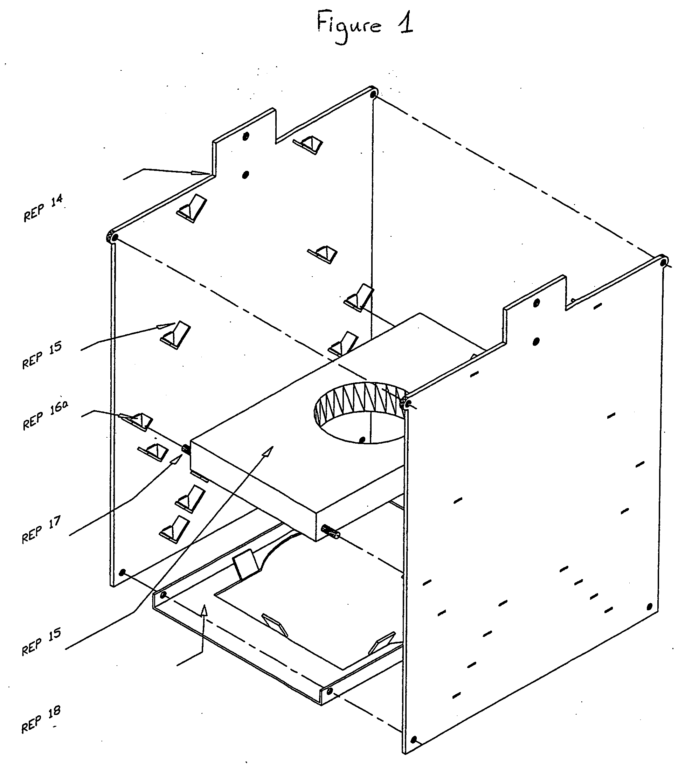

La figure 1 représente une vue du panier de masse en isométrique.

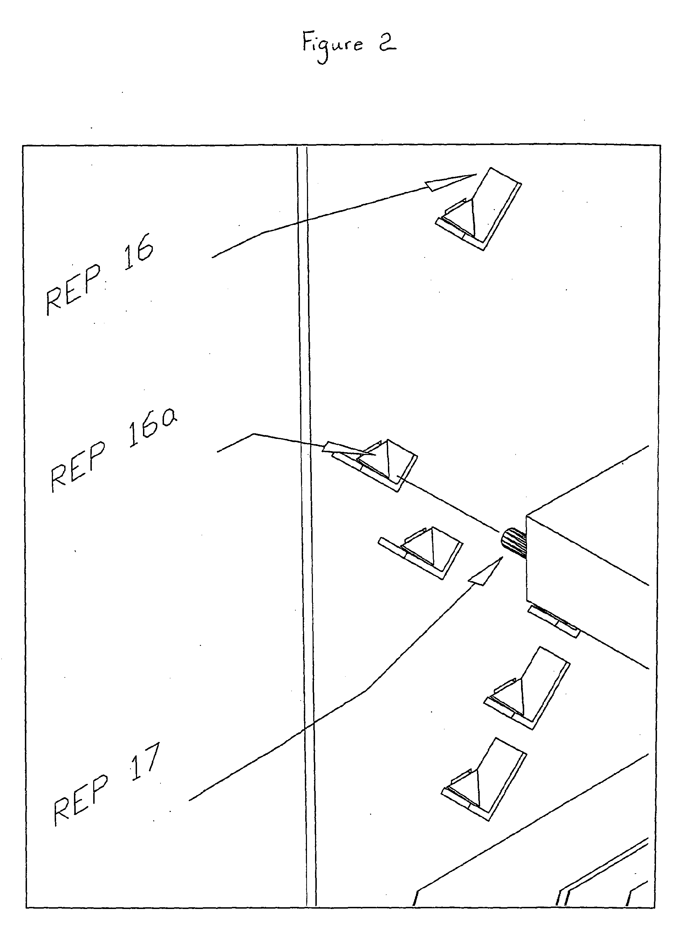

La figure 2 représente une vue de panier de masse en isométrique avec détail des supports de masse.

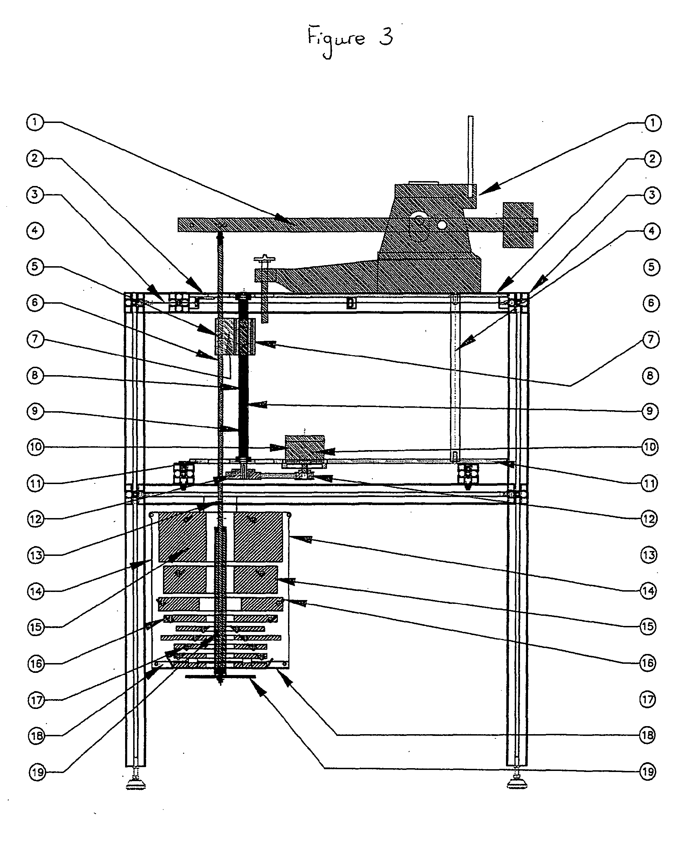

La figure 3 représente une vue de côté du plan d'ensemble.

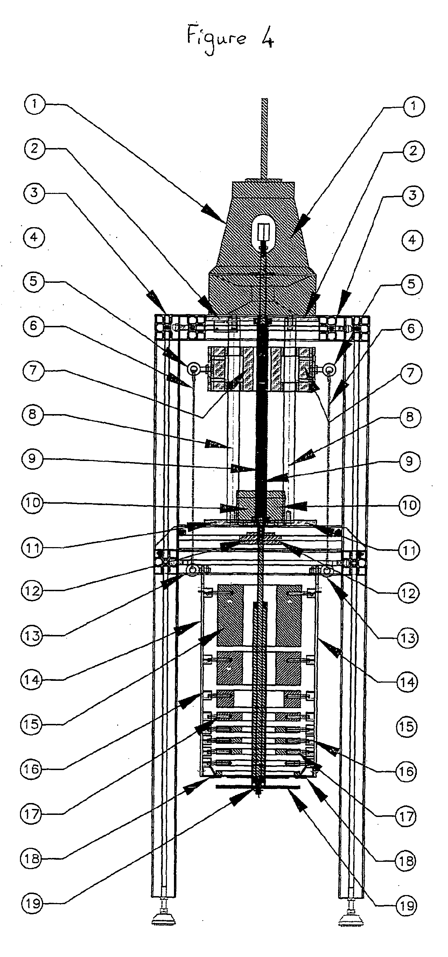

La figure 4 représente une vue de face du plan d'ensemble.

Chaque référence ci-dessous est en concordance avec le plan d'ensemble.
1. Oedomètre ( appareil ne faisant pas partie de ce brevet ) fixé sur le bâti supérieur (2)
2. Bâti supérieur sur féquel l'oedomètre est fixé ainsi que les axes (8) et (4) et qui maintient la vis sans fin (9)
3. Structure de la table ( ne fait pas partie du brevet ) , sur laquelle est fixé le bâti
   supérieur (2) et le bâti inférieur (11)
4. Axes de maintien entre le bâti supérieur (2) et inférieur (11)
5. oeillets supérieurs au nombre de deux , qui permettent de raccorder les câbles (6) au chariot mobile (7) et de suspendre le panier de masses (14)
6. Câbles de support du panier de masses (14)
7. Chariot mobile qui est mu par une vis sans fin (9) et qui glisse sur des axes (8) vers le haut et le bas
8. Axes parallèles sur lesquels glisse le chariot mobile (7)
9. Vis sans fin qui permet de faire descendre ou monter le chariot mobile (7), cette vis sans fin est mue par des poulies (12) et se raccorde par une courroie a un moteur électrique (10) ,elle est munie de roulement a billes en ses extrémités
10. Moteur électrique qui permet de faire déplacer le chariot mobile (7) vers le haut et le bas par l'intermédiaire des poulies (12) et d'une courroie
11. Bâti inférieur reposant sur la structure de la table (3), bâti sur lequel est fixé le moteur (10) ainsi que les axes (8) et (4) et qui maintien la base de la vis sans fin (9)
12. Poulies permettant de faire tourner la vis sans fin (9) afin de déplacer le chariot mobile (7) vers le haut ou le bas , les dites poulies sont entraînées par une courroie et le moteur (10)
13. oeillets inférieurs, qui permettent de raccorder les câbles (6) au chariot mobile (7) et de supporter le panier de masses (14)
14. Cotés du panier qui supportent les masses à déposer sur le plateau tige (19) et permettent de maintenir les masses en position levée grâce aux équerres (16) qui sont fixées sur les cotés internes du panier de masses. Les cotés du panier sont maintenus parallèle à l'aide de tiges dans sa partie supérieure et la platine (18) dans sa partie inférieure.
   Note : les équerres (16) du panier de masses sont disposées de façon a ce que les axes goupilles des masses ne puissent toucher les dites équ erres lors de la descente ou la remontée du panier de masses
15. Ensemble de masses qui sont au nombre de 9 selon la liste suivante :
   1. 1 fois 32 KG
   2. 1 fois 16 KG
   3. 1 fois 8 KG
   4. 1 fois 4 KG
   5. 1 fois 2 KG
   6. 1 fois 1 KG
   7. 1 fois 0.5 KG
   8. 1 fois 0.25 KG
   9. 1 fois 0.25 KG
   soit un ensemble total de 64 KG qui sont déposés sur le plateau tige (19)La valeur et le nombre de ces masses peut être modifiée selon l'évolution de la norme en vigueur se rapportant au principe d'essai de compressibilité des sols à l'oedomètre. Les masses sont percées d'un trou qui permet le passage de la tige du plateau tige (19)
16. Equerres disposées à 45 ° environ et fixées sur les cotés internes du panier support de masses (14) qui permettent de maintenir les huit premières masses (15) sur le panier de masses (14). Les équerres du panier de masse permettent de repositionner les masse dans le sens longitudinal lors de la reprise de ceux-ci par le papier de masses (14). Les équerres sont munies d'un triangle disposé en pente (16a) qui permet le recentrage des masses dans le sens latéral lors de la reprise de ceux-ci par le panier de masses (14).
17.Axes goupilles qui sont au nombre de quatre par masses, et fixées sur chaque masse servant d'axe d'accrochage afin de permettre aux masses d'être remontées lors de l'ascension du panier (14). Les axes goupilles permettent aux masses (15) de se maintenir sur le panier à l'aide des équerres (16) et des triangles de recentrage
18. Partie inférieure du panier de masses qui permet de recevoir la dernière masse de 0.25 kg qui ne dispose pas d'axe goupille, la partie inférieure du panier de masses (18) est découpée de façon à ce que le plateau tige (19) puisse passer au travers sans toucher le panier de masses (14) et la partie inférieure du panier de masses (18). Cette partie est fixée sur les cotés du panier de masse (14).
19. Ensemble plateau tige raccordé au bras de l'oedomètre, ce plateau solidaire de la tige reçoit les masses (15) lorsque le chariot mobile (7) descend et fait aussi descendre le panier de masses (14) permettant ainsi de déposer les masses (15) une par une sur le plateau tige (19) et de les remonter lors de la manoeuvre inverse de façon à décharger les masse du plateau tige (19) , la tige du plateau tige est renforcée dans sa partie inférieure pour l'empêcher de prendre une courbure et maintenir les masses en parfait émpilement.
   Le plateau tige (19) est destiné à recevoir les masses (15) de façon à créer une traction sur le plateau tige (19) qui est lui même raccordé par un axe au bras de l'oedomètre (1)

## Revendications

1. Dispositif de chargement automatique de masse pour oedomètre a chargement par l'avant **caractérisé en ce qu'**il comprend des moyens de chargement / déchargement de masses (15) empilées et logées dans un panier (14) sur un plateau (19) relié au bras de l'oedomètre

2. Dispositif selon la revendication 1 **caractérisé en ce que** les moyens de chargement/déchargement comprennent un corps (7) guidé par des tiges parallèle (8) en coulissement entraîné par une vis sans fin (9) et un moteur (10), le dit corps supportant le panier des masses (14) par des câbles (6) ou des tiges centrées par rapport a l'axe

3. Dispositif selon la revendication 2 **caractérisé en ce que** la plateau tige (19) traverse le panier (14) et les masses (15) de façon libre et en positio n centrée.

4. Dispositif selon l'une quelconque des revendications précédentes **caractérisé en ce qui** concerne le panier (14), comprend un arrangement de masses (15) , chaque masse présentant des protubérances latérales (17) , lesdites protubérances coopérant chacune avec des moyens de butée (16) notamment en forme évasée situées sur les cotés du panier de support des masses (14).

5. Dispositif selon la revendication 4 **caractérisé en ce que** lesdits moyens de butées (16) affectés a une première masse sont décalés horizontalement et verticalement par rapport au dit moyen de butée affectés a une seconde masse voisine haute ou basse afin de ne pas gêner l'empilement des masses (15)

6. Dispositif selon la revendication 4 ou 5 **caractérisé en ce que** chaque butée (16) présente une forme envasée à pans inclinés tridimensionnelles pour recentrer les masses (15) dans un plan donné lors du déchargement des masses par rapport à l'axe.

7. Dispositif selon l'une quelconque des revendications précédentes **caractérisé en ce que** le moteur (10) est commandé par un calculateur géré par un programme avec des fonctions d'empilement de masses (15) prédéterminées dans le temps et se rapportant à une norme ou à une méthode.
